(19) Europäisches Patentamt
European Patent Office
Office européen des brevets

(11) Numéro de publication: **0 466 586 B1**

(12) # FASCICULE DE BREVET EUROPEEN

(45) Date de publication de fascicule du brevet: **28.09.94**

(51) Int. Cl.⁵: **C07D 401/04**, A61K 31/53, C07D 401/12

(21) Numéro de dépôt: **91401916.1**

(22) Date de dépôt: **10.07.91**

(54) **Polyméthylène - imines disubstituées, leur procédé de préparation et les compositions pharmaceutiques les contenant.**

(30) Priorité: **11.07.90 FR 9008817**

(43) Date de publication de la demande:
**15.01.92 Bulletin 92/03**

(45) Mention de la délivrance du brevet:
**28.09.94 Bulletin 94/39**

(84) Etats contractants désignés:
**AT BE CH DE DK ES FR GB GR IT LI LU NL SE**

(56) Documents cités:
**EP-A- 0 090 733**

(73) Titulaire: **ADIR ET COMPAGNIE**
**1 rue Carle Hébert**
**F-92415 Courbevoie Cédex (FR)**

(72) Inventeur: **Regnier, Gilbert**
**Demeure du Plessis Bât. D,**
**27 avenue du Plessis**
**F-92290 Chatenay Malabry (FR)**
Inventeur: **Dhainaut, Alain**
**7 rue des Guipières**
**F-78400 Chatou (FR)**
Inventeur: **Atassi, Ghanem**
**4 rue Joséphine**
**F-92400 Saint Cloud (FR)**
Inventeur: **Pierre, Alain**
**52 rue de Montval**
**F-78160 Marly le Roi (FR)**
Inventeur: **Leonce, Stéphane**
**1 rue Lucis**
**F-78000 Versailles (FR)**

(74) Mandataire: **Reverbori, Marcelle**
**Adir et Compagnie,**
**1, rue Carle Hébert**
**F-92415 Courbevoie Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 466 586 B1

Rank Xerox (UK) Business Services
(3.10/3.09/3.3.3)

## Description

La présente invention a pour objet des polyméthylène-imines disubstituées, leur procédé de préparation et les compositions pharmaceutiques les renfermant.

- Elle concerne particulièrement les polyméthylène-imines de formule générale I :

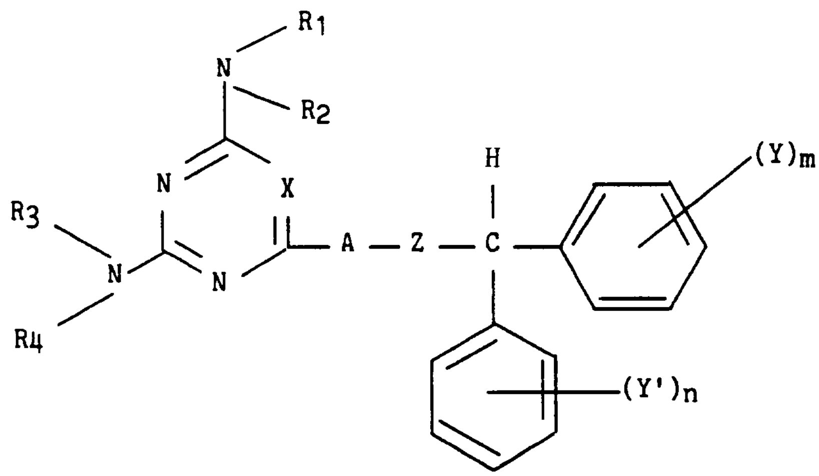

(I)

dans laquelle :

**a) X** représente le groupe CH ou un atome d'azote ;

**b) A** représente un groupe polyméthylène-imine de formule :

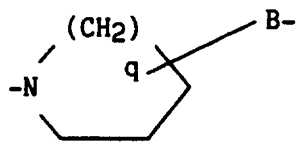

dans laquelle :

- **B** est un atome de soufre ou un radical NR' dans lequel R' représente un atome d'hydrogène ou un radical méthyle,
  et
  **q** est le nombre 2 ;

**c)** $R_1$, $R_2$, $R_3$, $R_4$ qui sont identiques ou différents, représentent chacun :

- un atome d'hydrogène, un radical allyle ou un radical propyle, étant entendu que l'un au moin des groupes

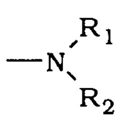

et

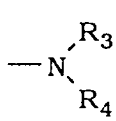

représente un radical allylamino ;

**d)** **Z** représente un radical méthylène ou éthylène ;

**e)** **Y** et **Y'**, qui sont identiques ou différents, représentent chacun un atome d'hydrogène ou de fluor ou un radical méthyle ou méthoxy ;

**f)** **m** et **n**, qui sont identiques ou différents, représentent chacun les nombres entiers 1 ou 2.

L'état antérieur de la technique est illustré notamment par le brevet français n° 2.524.467 qui a pour objet les polyméthylène-imines de formule :

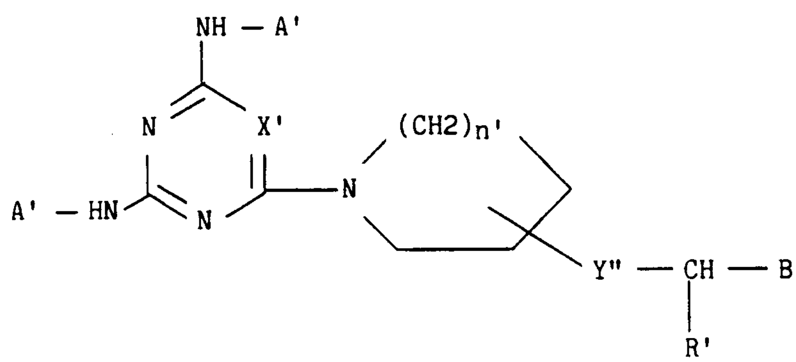

dans laquelle :

- **A'** est un radical alkényle de $C_3$ à $C_5$ éventuellement substitué par un ou plusieurs OH ;
- **X'** représente CH ou N ;
- **n'** représente zéro, un ou deux ;
- **Y"** représente 0 ou N-$R'_1$[$R'_1$ étant hydrogène, ($C_1$ - $C_5$) alkyle ou hydroxyalkyle, ($C_2$ - $C_5$) alkényle, ou ($C_3$ - $C_7$) - cycloalkyle ou cycloalkényle] ;
- **R'** est hydrogène, ($C_1$ - $C_5$) alkyle, ($C_5$ - $C_7$) cycloalkyle ou phényle éventuellement substitué,

et

- **B** est notamment ($C_1$ - $C_5$) alkyle, ($C_2$ - $C_5$) alkényle, phényle, naphtyle, benzofurannyle, benzothiényle, benzodioxolyle, benzodioxannyle, benzodioxinnyle, Δ3 chroményle, thio-chroményle ou chromanyle ;

lesquelles polyméthylène-imines favorisent la captation d'oxygène et peuvent ainsi être utilisées dans le traitement du déclin cérébral.

Des modifications importantes de structure ont conduit aux dérivés de formule I de la présente invention, lesquels présentent une activité pharmacologique et thérapeutique particulièrement intéressante et totalement differenciée de celle des dérivés proches antérieurement connus, comme le prouve l'étude pharmacologique décrite dans l'exemple 26.

- La présente invention a également pour objet le procédé de préparation des dérivés de formule générale I caractérisé en ce que l'on condense :
- soit une polyméthylène-imine de formule générale II :

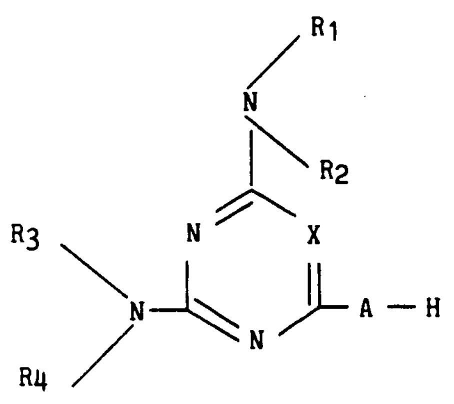

**(II)**

dans laquelle X, A, $R_1$, $R_2$, $R_3$ et $R_4$ ont les significations précédemment définies, sur un dérivé de formule

générale III :

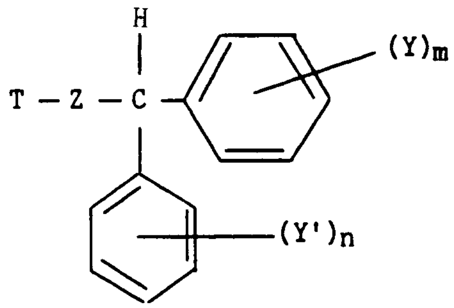

(III)

dans laquelle Z, Y, Y', m et n ont les significations précédemment définies et T représente un atome d'halogène tel que par exemple un atome de chlore ou de brome ou un reste tosyloxy

- soit un dérivé halogéné de formule générale IV :

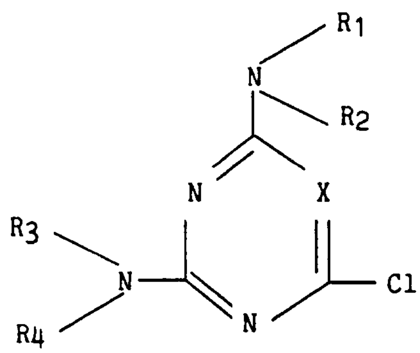

(IV)

dans laquelle X, $R_1$, $R_2$, $R_3$ et $R_4$ ont les significations précédemment définies, sur une polyméthylène-imine de formule générale V :

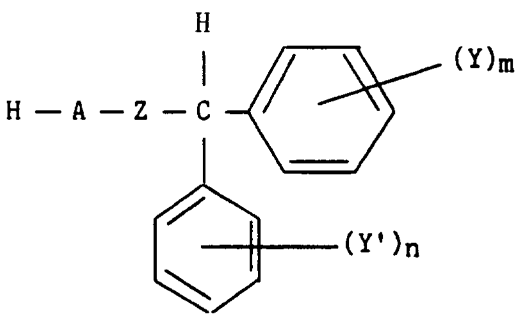

(V)

dans laquelle A, Z, Y, Y', m et n ont les significations précédemment définies.

- La condensation des dérivés II et III s'effectue de préférence dans un solvant choisi parmi les alcools contenant 4 ou 5 atomes de carbone, le diméthyl formamide, le déméthylacétamide, l'acétonitrile ou le tétrahydrofuranne.

  Il est avantageux d'opérer à une température comprise entre 80 et 120 °C en présence d'un accepteur de l'acide formé au cours de la réaction. Cet accepteur peut être choisi parmi les carbonates alcalins comme le carbonate de potassium, la triéthylamine ou un excès du dérivé II utilisé pour la condensation.

  D'autre part, dans le cas où B représente un atome d'oxygène ou de soufre (ce qui entraîne BH = OH ou SH) il est judicieux d'employer de l'hydrure de sodium pour soder préalablement le dérivé II.

- La condensation des dérivés IV et V s'effectue de façon particulièrement adéquate dans un solvant choisi parmi les alcools contenant 4 ou 5 atomes de carbone tel que le butanol ou le pentanol, et les

amides aliphatiques comme le diméthylformamide ou le diméthylacétamide. Il est recommandé d'opérer à une température comprise entre 120 et 150 °C en présence d'un accepteur de l'hydracide formé au cours de la réaction. Cet accepteur peut être choisi parmi les carbonates alcalins comme le carbonate de potassium, la triéthylamine ou un excès du dérivé V utilisé pour la condensation. Dans le cas où B représente un atome d'oxygène ou de soufre (ce qui entraine BH = OH ou SH) il est judicieux d'employer de l'hydrure de sodium pour soder le dérivé V.

La présente invention a aussi pour objet le procédé de préparation des dérivés I dans le cas où B représente le radical NR', c'est à dire plus spécifiquement des dérivés de formule générale I' :

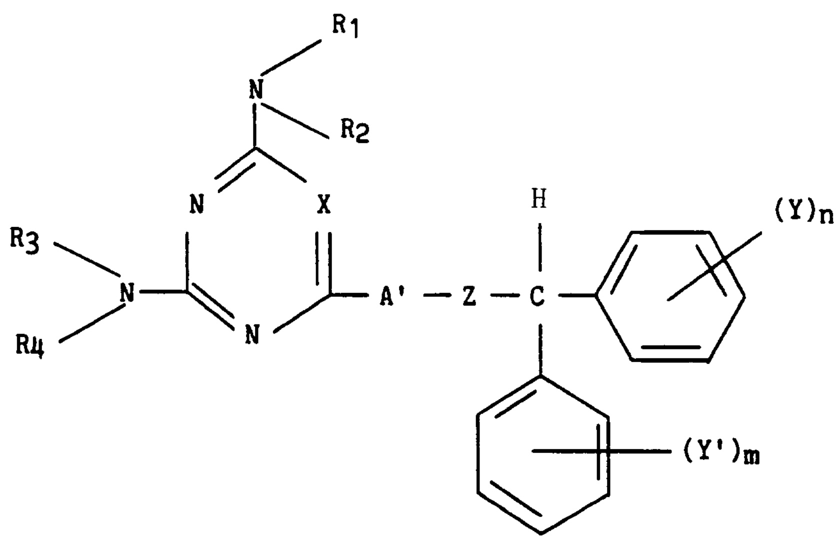

(I')

dans laquelle X, Z ,R, $R_1$, $R_2$, $R_3$, $R_4$, Y, Y', m et n ont les significations précédemment définies et A' représente un groupe polyméthylène-imine de formule :

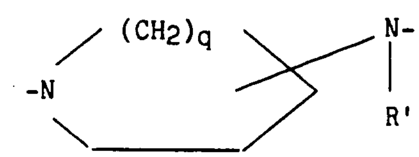

dans laquelle **q** et **R'** ont les significations précédemment définies.
caractérisé en ce que l'on traite par le cyanoborohydrure de sodium un mélange de :

- la cétone de formule générale VI :

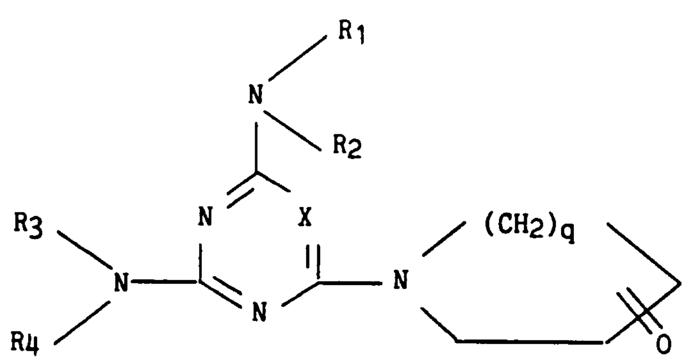

(VI)

dans laquelle X, $R_1$, $R_2$, $R_3$, $R_4$ et q ont les significations précédemment définies,

- et de l'amine de formule générale VII :

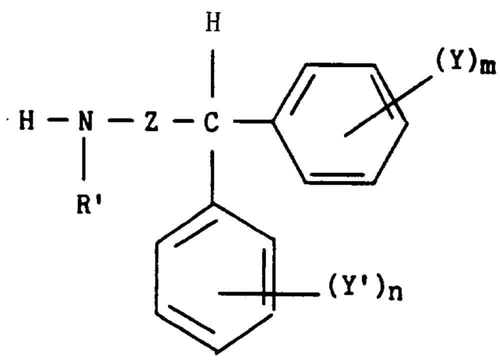

(VII)

dans laquelle R', Z, Y, Y', m et n ont les significations précédemment définies.

Il est particulièrement avantageux d'opérer dans un solvant approprié comme les alcools à bas poids moléculaire tel que par exemple le méthanol ou l'éthanol, ou le tétrahydrofuranne, à une température comprise entre 20 et 25 °C à un pH voisin de 6.

Les matières premières utilisées dans les procédés précédemment décrits sont soit des produits connus, soit des produits préparés à partir de substances connues, selon des procédés décrits pour préparer des produits analogues comme indiqué dans les exemples suivants.

Les dérivés de formule générale I peuvent être transformés en sels d'addition avec les acides, sels qui font, à ce titre, partie de l'invention. Comme acides utilisables pour la formation de ces sels, on peut citer, par exemple, dans la série minérale les acides chlorhydrique, bromhydrique, sulfurique, phosphorique et dans la série organique, les acides acétique, propionique, maléique, fumarique, tartrique, nitrique, oxalique, benzoïque, méthane sulfonique et iséthionique.

D'autre part, dans le cas où Z est une chaine ramifiée et ou Y et Y' sont différents l'un de l'autre et différents d'un atome d'hydrogène, les dérivés (I) peuvent se présenter sous forme de diastéréoisomères ou d'énantiomères qui font, à ce titre, également partie de l'invention. Les nouveaux dérivés (I) peuvent être purifiés par des méthodes physiques telles que cristallisation des bases, chromatographie (en particulier chromatoflash sur silice 35-70$\mu$, sous une pression de 0,5 à 1 atmosphère d'azote, avec comme système d'élution : $CH_2$ $Cl_2$ - méthanol ou acétate d'éthyle), ou chimiques telles que formation de sels d'addition avec des acides et décomposition de ces sels par des agents alcalins.

Les dérivés de formule générale I et leurs sels d'addition physiologiquement tolérables possèdent des propriétés pharmacologiques et thérapeutiques intéressantes, qui permettent de les utiliser pour supprimer la résistance des cellules tumorales aux agents anti-cancéreux et pour supprimer la résistance des parasites aux agents anti-parasitaires.

La présente invention a également pour objet les compositions pharmaceutiques contenant comme principe actif, un dérivé de formule générale I ou un de ses sels physiologiquement tolérable, mélangé ou associé à un excipient pharmaceutique approprié.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par la voie orale, rectale ou parentérale.

La posologie peut varier notablement selon l'âge et le poids du patient, la voie d'administration, la nature de la maladie et les traitements associés et s'échelonne de 0,1 à 7 g par prise.

Les exemples suivants illustrent l'invention. Les points de fusion sont déterminés au tube capillaire (cap) ou à la platine chauffante de kofler (K).

**Exemple 1 :**

(bis allylamino-4,6 s.triazinyl-2)-1[(bis p.fluorophényl)-2,2 éthyl] amino-4 pipéridine.

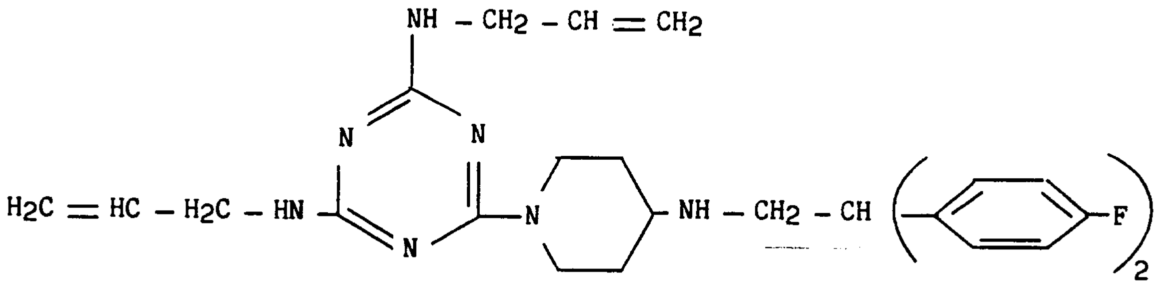

**A) Première méthode :**

A une solution de 4,6 g de cyanoborohydrure de sodium dans 150ml de méthanol contenant 20 g de tamis moléculaire 3Å, on ajoute successivement, à une température de 15 à 20°C, 17 g de bis-(p.fluorophényl)-2,2 éthylamine ($Eb/_{7mm}$ = 158-160°C) puis 23,7 g de chlorhydrate de (bis allylamino -4,6 triazinyl-2)-1 pipéridone -4, fondant (cap) à 219-222°C.

On ajuste de pH à 6 par addition de méthanol chlorhydrique et laisse agiter pendant 24 heures à température ambiante.

Au bout de ce temps, on filtre l'insoluble et évapore le filtrat sous pression réduite.

On reprend le résidu par de l'éther, lave à l'eau puis avec une solution à 10 % de Na $HCO_3$.

On sèche sur Mg $SO_4$. Après évaporation, le résidu est chromatographié sur $SiO_2$ en éluant avec $CH_3$ COO $C_2H_5$. Après évaporation des éluats, on recristallise le résidu critallin dans l'éthanol à 90 %. On obtient 5,3g de monohydrate fondant (cap) à 66-68°C.

Le chlorhydrate de bis(allylamino-4,6 triazinyl-2)-1 pipéridone-4 de départ, a été préparé par condensation de la diallylamino-4,6 chloro-2 triazine avec la diéthoxy-4,4 pipéridine, puis hydrolyse du dérivé diéthoxylé ainsi obtenu, au moyen d'HCl dilué, en pipéridone correspondante.

**B) Deuxième méthode :**

On chauffe pendant 8 heures à 120°C une solution de 2,9 g de bis allylamino -4,6(amino-4 pipéridino)-2 triazine - 1, 3, 5, fondant (cap) à 120°C et de 3g de (bis p.fluorophényl)-2,2 bromo-1 éthane dans 50ml de diméthylformamide, en présence de 1,2 g de triéthylamine.

Lorsque la réaction est terminée, on évapore le solvant sous pression réduite et le résidu est repris dans l'éther puis lavé à l'eau. Après évaporation de l'éther, le résidu huileux est chromatographié sur $SiO_2$ en éluant avec $CH_3$ COO $C_2H_5$.

L'évaporation des éluats laisse un résidu cristallin que l'on cristallise dans l'éthanol à 90 %. On obtient 1,9 g de cristaux de monohydrate fondant (cap) à 66-68°C.

La bis allylamino-4,6 (amino-4 pipéridino)-2 triazine-1, 3, 5 de départ a été préparée par condensation de l'acétamido-4 pipéridine avec la bis allylamino-4,6 chloro-2 triazine dans le diméthylformamide, suivie d'une hydrolyse du composé résultant au moyen d'une solution d'hydroxyde de sodium dans l'éthanol.

**Exemple 2 :**

(bis allylamino-4,6 s.triazinyl-2)-1 (diphényl-3,3 propyl)thio-4 pipéridine.

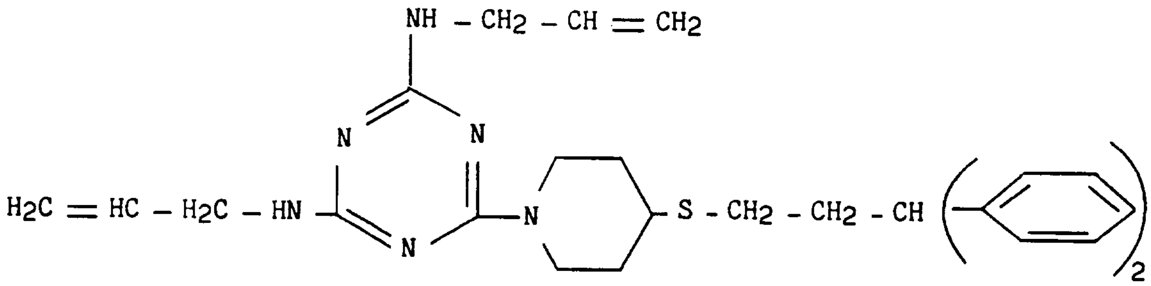

On chauffe à reflux, pendant 10 heures, une solution de 3,6 g de bis allylamino-4,6 chloro-2 triazine et de 5 g de (diphényl-3,3 propylthio)-4 pipéridine dans 100ml de butanol, en présence de 4,4 g de $K_2 CO_3$. Lorsque la réaction est terminée, on filtre le sel et évapore le solvant sous pression réduite. On reprend le résidu par l'éther. On obtient 9 g d'huile dont le fumarate, préparé au sein de l'éthanol donne 7,8 g de cristaux fondant (K) à 168 °C.

La pipéridine de départ, dont le fumarate fond (K) à 160 °C, a été préparée par hydrolyse, au moyen de $(CH_3)_3$ SiCl, du dérivé N-carbéthoxylé correspondant.

**Exemple 3 :**

(bis allylamino-4,6 s.triazinyl-2) amino-4 (diphényl-2,2 éthyl)-1 pipéridine.

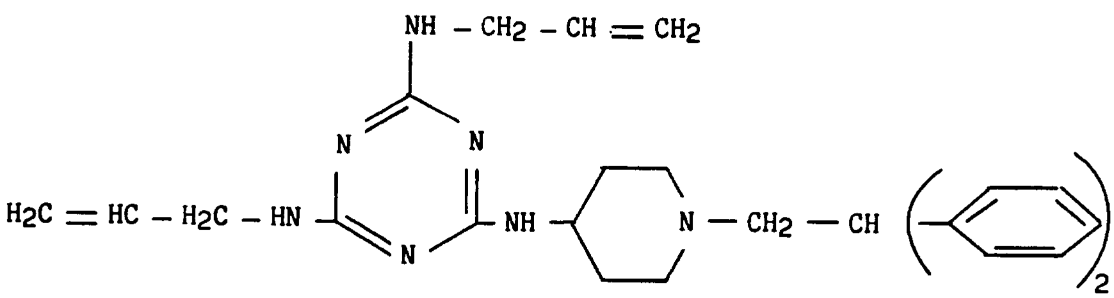

On chauffe, pendant 10 heures, à reflux, une solution de 2,3 g de diallylamino-4,6 chloro-2 triazine- 1, 3, 5 et de 2,8 g d'amino-4 (diphényl-2,2 éthyl)-1 pipéridine en présence de 1,4 ml de triéthylamine, dans 50 ml de butanol. Lorsque la réaction est terminée, on évapore le butanol sous pression réduite, et reprend le résidu avec de l'éther.

La solution éthérée est lavée à l'eau puis l'éther est évaporé. Le résidu huileux est purifié par chromatographie sur $SiO_2$ en éluant avec $CH_3$ COO $C_2H_5$.

Après évaporation des éluats, on obtient 2,6 g d'un produit sous forme de résine.

L'amino-4(diphényl-2,2 éthyl)-1 pipéridine de départ, P.F.(cap):41-45 °C, a été préparée par réduction alkylative, au moyen de $NaBH_3CN$ dans le méthanol, d'un mélange de diphénylacétaldéhyde et d'acétamido-4 pipéridine, suivie d'une hydrolyse, avec HCl 4N, de la diphényl-éthyl-1 acétamido-4 pipéridine formée.

**Exemple 4 :**

(bis allylamino-4,6 s.triazinyl-2)-1[N-(diphényl-2,2 éthyl) N-méthylamino]-4 pipéridine.

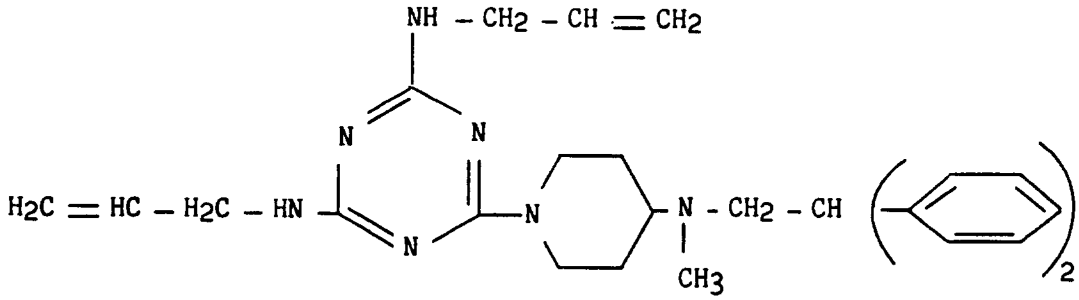

On chauffe pendant 8 heures à 130 ° C une solution de 2,3 g de diallylamino-4,6 chloro-2 triazine-1, 3, 5, fondant (cap) à 206-208 ° C, et de 3 g de [N-(diphényl-2,2 éthyl) N-méthylamino]-4 pipéridine dans 50 ml de diméthylformamide, en présence de 1,4 g de $K_2CO_3$. Lorsque la réaction est terminée, on filtre le sel et évapore le solvant sous pression réduite.

Le résidu est chromatographié sur Si $O_2$ en éluant avec $CH_2Cl_2$-$CH_3OH$( 95-5).

On obtient 3,2 g d'un produit huileux, dont le difumarate fond (cap) à 172-176 ° C.

La pipéridine de départ (huile) a été préparée par réduction alkylative, au moyen de $NaBH_3CN$ dans le méthanol, de l'acétyl-1 pipéridone-4 avec la N-(diphényl-2,2 éthyl) N-méthyl amine[P.F.(cap) de l'oxalate = 215 ° C] suivie d'une hydrolyse alcaline avec Na OH-$C_2H_5OH$.

La (bis allylamino-4,6 s.triazinyl-2)-1[N-(diphényl éthyl)N-méthyl amino]-4 pipéridine a également été préparée selon le procédé décrit dans l'exemple 1 méthode B).

**Exemples 5 à 28 :**

En appliquant un ou plusieurs des procédés de préparation décrits dans les exemples 1 à 4, ont été préparés les dérivés suivants :

**5)** (bis allylamino-4,6 s.triazinyl-2)-1[(diphényl-2,2 éthyl)amino]-4 pipéridine, P.F.(cap) : 69-72 ° C.

**6)** (bis allylamino-4,6 s.triazinyl-2)-1[(diphényl-3,3 propyl)amino]-4 pipéridine, P.F.(cap) : 88-92 ° C.

**7)** (propylamino-4 allylamino-6 s.triazinyl-2)-1[(diphényl-2,2 éthyl) amino]-4 pipéridine, P.F.(cap) : 56-58 ° C.

**8)** (propylamino-4 allylamino-6 s.triazinyl-2)-1[(diphényl-3,3 propyl) amino]-4 pipéridine, P.F.(cap) : 78-83 ° C.

**9)** (bis allylamino-2,4 pyrimidinyl-6)-1[(diphényl-3,3 propyl)amino]-4 pipéridine, P.F.(cap) du fumarate : 209-211 ° C.

**10)** (bis allylamino-4,6 s.triazinyl-2)-1[(diméthoxy-3,5 phényl)-2(méthyl-2 phényl)-2 éthyl amino]-4 pipéridine, P.F.(cap) du difumarate : 178-184 ° C.

**11)** [(allylamino-4 propylamino-6 s.triazinyl-2)amino]-4 (diphényl-2,2 éthyl)-1 pipéridine.

**12)** (bis allylamino-4,6 s.triazinyl-2)-1[bis(diméthoxy-3,4 phényl)-3,3 propyl amino]-4 pipéridine, P.F. (cap) du fumarate : 180-182 ° C.

**Exemple 13 :**

**Etude pharmacologique**

La résistance aux agents anticancéreux est un obstacle majeur à l'efficacité des drogues antitumorales. Les cellules tumorales lorsqu' elles sont exposées in vitro ou in vivo à un agent anticancéreux deviennent résistantes, à divers degrés à ces composés.

De nombreux mécanismes d'acquisition par une cellule de résistance aux agents anticancéreux ont été décrits. Parmi les différents types de résistance, la "Multidrug Resistance" (MDR) est particulièrement intéressante. Le phénomène de résistance est dû à l'action d'une protéine membranaire inductible, la gP 170, dont le rôle est d'augmenter l'efflux du cytotoxique, donc de diminuer sa concentration intracellulaire,

d'où la perte de sensibilité de ces cellules à la drogue.

Des médicaments, utilisés dans d'autres pathologies, sont connus pour reverser, partiellement ou totalement cette résistance (Int. J. Cancer Res. (1988) +9 pp 285-296 ; I.N.C.I. (1989) 81 PP 907-910 ; Trends Pharmacol Sci (1989) 9 pp 54-58 ; Annu. Rev. Biochem. (1989) 58 pp 137-171.

L'agent modulateur, lorsqu' il est ajouté en même temps que le cytotoxique, diminue ou supprime totalement la résistance de type MDR. Certains médicaments utilisés pour le traitement d'autres maladies comme l'amiodarone, le verapamil ou la ciclosporine, ont été utilisés en clinique pour lever cette résistance, mais leurs propriétés pharmacologiques intrinsèques (agents hypotenseurs, ou immunosuppresseurs) souvent indésirables lors du traitement du cancer et leurs toxicités, limitent considérablement leur utilisation.

De plus le mécanisme de la résistance à la chloroquine, développée par le Plasmodium falciparum est similaire. Le verapamil restaure la sensibilité d'une lignée résistante, ce qui démontre l'intérêt potentiel de composés reversant le phénotype MDR des cellules tumorales, pour une utilisation en parasitologie. (Science (1987), 238, pp 1283-1285 ; Science (1987), 235, pp 899-901).

Les tests ci-après décrits montrent que les dérivés de la présente invention réversent la résistance acquise aux différents médicaments.

**A) Evaluation de l'augmentation de la cytotoxicité de l'adriamycine sur la lignée P 388/ADR-10 in vitro**

Lors de cette étude, on a mesuré la cytotoxicité de l'adriamycine, en absence et en présence du composé reversant. Pour cet essai, on a utilisé la leucémie murine P 388/ADR-10 dont la résistance a été induite par l'adriamycine. Son facteur de résistance est de 260 par rapport à la lignée sensible (résistance moyenne).

Les cellules sont cultivées dans un milieu de culture (RPMI 1640) complet, contenant 10 % de serum de veau foetal, 2 nM de glutamine, 50 UI/ml de pénicilline, 50 µg/ml de streptomycine, 10 mM de Hepes et 20 nM de bêta-mercaptoéthanol.

Les cellules sont réparties dans des microplaques et exposées à l'adriamycine à 9 concentrations différentes.

Les produits testés pour leur capacité à reverser la MDR sont ajoutés en même temps que le cytotoxique. Les cellules sont ensuite incubées pendant 48 heures.

Le nombre de cellules viables est ensuite quantifié par un essai colorimétrique, le Microculture Tetrazolium Assay (Cancer Res. (1987), 47, pp 936-942).

Les résultats sont exprimés en $IC_{50}$, concentration en cytotoxique qui inhibe à 50 % la prolifération des cellules témoins. Les résultats sont exprimés en Facteur de Reversion (FR).

$$FR = \frac{IC_{50} \text{ Cytotoxique seul}}{IC_{50} \text{ Cytotoxique en présence du composé reversant}}$$

Le tableau I donne les valeurs des facteurs de reversions obtenus avec les différents composés de formule I et les produits de référence, et démontre l'activité très intéressante des composés de formule I.

Concernant la reserpine (un des produits de référence), ce composé a une très bonne activité in vitro mais in vivo, il est inutilisable à cause de sa grande toxicité.

TABLEAU I

| PRODUITS | 2,5 µM | 5 µM | 10 µM |
|---|---|---|---|
| **PRODUITS DE REFERENCE** | | | |
| PHENOTHIAZINE | 9.4 | 23.1 | - |
| PROGESTERONE | - | 1.7 | 2.5 |
| FLUNARIZINE | - | - | 25 |
| QUINIDINE | - | 6 | 17.4 |
| QUININE | - | - | 14.5 |
| VERAPAMIL | 13.5 | 36.3 | 67.2 |
| AMIODARONE | 37.7 | 104.7 | TOX |
| RESERPINE | - | 215.5 | 189 |
| CICLOSPORINE | 149.4 | TOX | TOX |
| **PRODUITS DES EXEMPLES** | | | |
| EXEMPLE 1 | 110.8 | 244.4 | 170.7 |
| EXEMPLE 2 | 15.4 | 65.1 | 86.4 |
| EXEMPLE 3 | 39 | 70 | 134.8 |
| EXEMPLE 4 | 34.7 | 32.3 | 171.1 |
| EXEMPLE 5 | 100 | 189 | 277.4 |
| EXEMPLE 6 | 54.4 | TOX | TOX |
| EXEMPLE 7 | 102.8 | 135.6 | TOX |
| EXEMPLE 8 | 75.9 | TOX | TOX |
| EXEMPLE 9 | 80.4 | TOX | TOX |
| EXEMPLE 10 | 125.1 | 75.3 | TOX |
| EXEMPLE 11 | 87 | 65.2 | TOX |
| EXEMPLE 12 | 1.4 | 8 | TOX |

**B) Evaluation de l 'augmentation de la cytotoxicité de l 'actinomycine D sur la lignée de poumon de hamster chinois. DC-3F/AD**

Le protocole utilisé pour cette étude est identique à celui utilisé pour l'essai décrit dans l'exemple 1 mais le milieu de culture ne contenait pas de bêta-mercaptoéthanol et les cellules ont été incubées pendant 4 jours au lieu de 48 heures. Le cytotoxique utilisé était l'actinomycine D.

La lignée DC-3F/AD est une lignée extrémement résistante. Son facteur de résistance est supérieur à 10.000.

Les résultats de cette étude sont donnés dans le tableau II :

Les résultats du tableaux II démontrent que les composés de formule I diminuent de manière significative ou suppriment la résistance au cytotoxique.

TABLEAU II

| PRODUITS | 2,5 µM | 5 µM | 10 µM | 20 µM |
|---|---|---|---|---|
| **PRODUITS DE REFERENCE** | | | | |
| PHENOTHIAZINE | --- | < 12 | < 12 | < 12 |
| CHLOROPROMAZINE | --- | < 13 | < 13 | < 13 |
| YOHIMBINE | --- | < 12 | < 12 | < 12 |
| NIFEDIPINE | --- | < 11 | TOX | TOX |
| PROGESTERONE | --- | < 10 | < 10 | 13 |
| QUININE | --- | < 10 | < 10 | < 11 |
| DILTRIAZEM | --- | < 11 | < 10 | < 11 |
| FLUNARIZINE | --- | < 13 | 16 | TOX |
| DIPYRIDAMOLE | --- | < 12 | < 11 | < 12 |
| QUINIDINE | --- | < 12 | < 12 | < 12 |
| QUINACRINE | --- | < 13 | < 12 | TOX |
| TRIFLUOPERAZINE | --- | < 12 | 38 | TOX |
| VERAPAMIL | --- | < 13 | 31 | 117 |
| AMIODARONE | --- | 298 | 388 | TOX |
| PIMOZIDE | < 10 | 28 | 78 | TOX |
| RESERPINE | 258 | 1085 | 1649 | 2024 |
| CICLOSPORINE | < 11 | < 11 | 11 | 10 |
| **PRODUITS DES EXEMPLES** | | | | |
| EXEMPLE 1 | 171 | 905 | 2575 | TOX |
| EXEMPLE 2 | - | 17 | 171 | - |
| EXEMPLE 3 | 38 | 104 | 240 | - |
| EXEMPLE 4 | - | 218 | 1256 | - |
| EXEMPLE 5 | - | 658 | 1504 | TOX |
| EXEMPLE 6 | < 16.5 | 220 | TOX | TOX |
| EXEMPLE 7 | - | 992 | 2339 | - |
| EXEMPLE 8 | 16.5 | TOX | TOX | TOX |
| EXEMPLE 9 | 94 | 472 | TOX | - |
| EXEMPLE 10 | 259 | 1347 | TOX | - |
| EXEMPLE 11 | - | 281 | 926 | - |
| EXEMPLE 12 | - | < 16.5 | < 16.5 | - |

**C) Cytométrie en flux**

Certains composés anticancéreux comme l'adriamycine (ADR) ont la propriété d'être fluorescents après excitation par une source lumineuse de longueur d'onde connue.

Par la mesure de cette fluorescence, il est possible de mesurer de façon relative la concentration intracellulaire en ADR. La cytométrie en flux (CMF) est un outil de choix pour effectuer ce type de mesure et ainsi déterminer rapidement si certains composés actifs agissent en augmentant la concentration intracellulaire en adriamycine.

Les cellules (500.10[3)] par ml ont été exposées simultanément à l'adriamycine à une concentration fixe (50 µM) et aux composés testés, aux concentrations de 2,5, 10 et 20 µM. Après 5 heures d'incubation, l'uptake de l'adriamycine intra-cellulaire a été évalué par CMF.

Les analyses ont été réalisées sur un cytomètre en flux ATC 3000 (Bruker - France) équipé d'un laser argon 2025 (Spectra-Physics-France®) optimisé à 488 nm pour une puissance de 600 mW.

L'analyse de chacun des échantillons a été effectuée sur un total de 10.000 cellules à une vitesse de 1000 cellules/sec.

Les résultats ont été collectés sous forme d'histogrammes linéaires de la fluorescence de l'ADR intra-cellulaire.

**Expression des résultats :**

Pour chacun des histogrammes, le canal moyen (MEAN) de fluorescence a été déterminé par le système informatique de l'appareil.
Pour toutes expériences :

- Un contrôle négatif (cellules sans ADR) a fixé le seuil d'auto-fluorescence.
- Un contrôle positif (cellules avec ADR) a déterminé la valeur MEAN = MN1.
- Les tubes "tests" (cellules avec ADR et avec produit) ont déterminé pour chacun des produits et à chacune des concentrations, les valeurs MEAN = MN2.
- Les résultats sont exprimés sous forme de variation de la moyenne de fluorescence obtenue pour chacun des tubes "tests" (MN2) par rapport à la moyenne de la fluorescence obtenue avec le contrôle positif (MN1) : VAR-MEAN = MN2 - MN1 . Le paramètre exprimé est donc l'augmentation de la fluorescence de l'adriamycine en présence des composés testés.

Le tableau III donne l'augmentation de la fluorescence de l'ADR obtenue avec les différents composés sur la lignée DC-3F/AD et le tableau IV sur la lignée P 388/ADR-10.

TABLEAU III

| **PRODUITS** | **2 μM** | **5 μM** | **10 μM** | **20 μM** |
|---|---|---|---|---|
| **PRODUITS DE REFERENCE** | | | | |
| VERAPAMIL | 2.00 | 2.60 | 7.55 | 11.45 |
| AMIODARONE | 8.80 | 16.65 | 21.55 | 26.35 |
| PIMOZIDE | 5.85 | 8.85 | 15.65 | 21.60 |
| RESERPINE | 26.18 | 29.43 | 31.73 | 29.95 |
| CICLOSPORINE | 1.90 | 3.20 | 8.05 | 15.10 |
| QUINACRINE | 2.00 | 10.5 | 19.1 | 25.8 |
| TRIFLUOPERAZINE | 0.00 | 0.00 | 4.10 | 13.40 |
| **PRODUIT DES EXEMPLES** | | | | |
| EXEMPLE 1 | 18.27 | 26.13 | 35.57 | 43.63 |
| EXEMPLE 2 | 0.2 | 10.4 | 25.1 | 35.0 |
| EXEMPLE 3 | 8.0 | 19.9 | 21.6 | 19.3 |
| EXEMPLE 4 | 12.3 | 24.1 | 41.9 | 49.0 |
| EXEMPLE 5 | 15.5 | 31.3 | 34.65 | 37.9 |
| EXEMPLE 6 | 3.9 | 21.5 | 29.75 | TOX |
| EXEMPLE 7 | 14.47 | 26.0 | 34.17 | 36.63 |
| EXEMPLE 8 | 4.5 | 17.85 | 27.15 | TOX |
| EXEMPLE 9 | 7.2 | 15.1 | 16.3 | 26.2 |
| EXEMPLE 10 | 14.8 | 24.7 | 26.5 | 28.2 |
| EXEMPLE 11 | 12.6 | 18.9 | 29.9 | 40.5 |
| EXEMPLE 12 | 6.1 | 3.8 | 5.5 | 9.6 |

TABLEAU IV

| PRODUITS | 2 µM | 5 µM | 10 µM | 20 µM |
|---|---|---|---|---|
| **PRODUITS DE REFERENCE** | | | | |
| PHENOTHIAZINE | 0,00 | 0,00 | 0,00 | 0,00 |
| CHLOROPROMAZINE | 3,30 | 5,15 | 4,60 | 6,20 |
| YOHIMBINE | 2,30 | 0,05 | 4,70 | 3,45 |
| NIFEDIPINE | 0,00 | 0,80 | 5,95 | 3,40 |
| PROGESTERONE | 1,85 | 1,00 | 6,85 | 11,80 |
| QUININE | 4,85 | 5,40 | 12,00 | 18,90 |
| DILTIAZEM | 3,10 | 6,65 | 13,45 | 23,15 |
| FLUNARIZINE | 7,35 | 10,10 | 19,90 | 41,00 |
| DIPYRIDAMOLE | 2,35 | 7,55 | 21,75 | 40,00 |
| QUINIDINE | 6,65 | 12,80 | 22,00 | 29,90 |
| QUINACRINE | 11,83 | 15,73 | 27,73 | 52,77 |
| TRIFLUOPERAZINE | 11,45 | 14,75 | 34,55 | 51,05 |
| VERAPAMIL | 9,89 | 21,90 | 39,62 | 56,50 |
| AMIODARONE | 33,40 | 65,25 | 76,33 | 95,85 |
| PIMOZIDE | 25,60 | 49,42 | 73,13 | 73,64 |
| RESERPINE | 73,46 | 80,48 | 91,90 | 85,39 |
| CICLOSPORINE | 79,43 | 97,63 | 96,68 | 90,27 |
| **PRODUITS DES EXEMPLES** | | | | |
| EXEMPLE 1 | 53.1 | 74.7 | 85.49 | 78.5 |
| EXEMPLE 3 | 29.9 | 46.2 | 66.0 | 73.1 |
| EXEMPLE 5 | 45.25 | 60.7 | 79.47 | 76.25 |
| EXEMPLE 6 | 32.9 | 60.1 | 55.8 | TOX |
| EXEMPLE 7 | 48.75 | 72.6 | 80.9 | 87.3 |
| EXEMPLE 8 | 38.9 | 60.1 | TOX | TOX |
| EXEMPLE 9 | 39.2 | 55.8 | 65.8 | TOX |
| EXEMPLE 10 | 52.0 | 75.3 | 84.0 | 78.0 |

**D) Réversion de la résistance acquise aux médicaments, in vivo**

Le produit de l'exemple 1 a été testé sur une tumeur présentant le phénotype MDR, la leucémie murine P388/ADR. Plusieurs rapports de doses du produit de l'exemple 1 et d'adriamycine ont été utilisés.

La leucémie P388/ADR (résistante à l'ADR) provient du National Cancer Institute (USA).

$10^6$ cellules sont greffées par voie intrapéritonéale aux souris $B_6D_2F_1$ femelles au jour 0 et le traitement commence le lendemain selon les schémas d'administration indiqués. Le produit de l'exemple 1 est préparé dans l'hydroxypropyl cellulose, la suspension est ensuite homogénéisée à l'Ultra Turrax. Le produit de l'exemple 1 est administré par voie IP 30 à 60 minutes avant le cytotoxique (0,2 ml dans les deux cas).

Le paramètre mesuré est le temps de survie qui permet de calculer le T/C :

$$T/C = \frac{\text{Temps médian de survie des animaux traités}}{\text{Temps médian de survie des animaux témoins}} \times 100$$

La variation pondérale est calculée, elle renseigne sur la toxicité du traitement. Les groupes témoins comportent de 15 à 27 animaux, et les groupes traités de 5 à 10 animaux.

Six expériences indépendantes ont été réalisées. Les essais ont été effectués sur 4 jours selon le schéma $J_{1-4}$ : les cellules tumorales étant greffées à $J_0$ et le réversant et le cytotoxique administrés à $J_1$, $J_2$, $J_3$ et $J_4$.

Les résultats obtenus sont répertoriés dans le tableau V sous forme de résultats globaux (T/C moyens et médians). La lignée P388 est bien résistante, l'ADR seule ne présentant aucune activité antitumorale (T/C<120 %).

Le produit de l'exemple 1, testé de 50 à 200 mg/kg, ne possède pas d'activité antitumorale et ne présente qu'une toxicité modérée à 200 mg/kg.

Une activité modeste est obtenue avec le produit de l'exemple 1 à 50 et 100 mg/kg (120 %<T/C<135 %) et une bonne activité est obtenue avec 200 mg/kg de produit de l'exemple 1 et 2 mg/kg d'ADR (T/C = 148,9 %). Dans ce cas, le gain d'activité dû au produit de l'exemple 1 est de 30,6 %. De plus, le tableau V montre que l'activité est reproductible, les T/C moyens étant très proches des T/C médians lorsque le nombre d'expériences indépendantes prises en compte est de quatre ou six.

Tableau V

Lignée P388/ADR Cytotoxique ADR Schéma $J_{1-4}$ (QDX4,1-4)

Administrations intrapéritonéales, T/C moyens et médians

| Composés | | n1 | *T/C Valeurs extrêmes | *T/C Moyenne ±e.s.m. | *T/C Médiane |
|---|---|---|---|---|---|
| TEMOINS | | 6 | 100 | 100 | 100 |
| Exemple 1 | 50 mg/kg | 2 | 90 - 108,3 | 99,2 | 99,2 |
| " | 100 mg/kg | 6 | 85 - 100 | 91,7 ± 2,8 | 90 |
| " | 200 mg/kg | 4 | 80 - 88,9 | 83,5 ± 2,2 | 82,5 |
| ADR | 2 mg/kg | 2 | 116,7 - 120 | 118,3 | 118,3 |
| " | 4 mg/kg | 6 | 105 - 130 | 115,1 ± 3,9 | 112,5 |
| Exemple 1 | 50 mg/kg | | | | |
| + ADR | 2 mg/kg | 2 | 120 - 141,7 | 130,9 | 130,9 |
| + ADR | 4 mg/kg | 2 | 116,7 - 140 | 128,4 | 128,4 |
| Exemple 1 | 100 mg/kg | | | | |
| + ADR | 2 mg/kg | 2 | 116,7 - 140 | 128,4 | 128,4 |
| + ADR | 4 mg/kg | 6 | 120 - 150 | 134,2 ± 4,4 | 134,2 |
| Exemple 1 | 200 mg/kg | | | | |
| + ADR | 2 mg/kg | 4 | 140 - 155,6 | 148,9 ± 3,2 | 150 |

Les résultats sont exprimés en T/C (%) qui est le rapport des temps médians de survie des animaux traités/animaux témoins

*T/C ≤ 85 % toxicité T/C ≥ 120 % : Activité modérée

T/C ≥ 135 % : Bonne activité (critère de sélection d'un produit). T/C ≥ 175 % : Activité importante

n1 = nombre d'expériences indépendantes : nombre de valeurs prises en compte pour le calcul des T/C moyens et médians.

## Revendications

**1.** Les polyméthylène-imines de formule générale I :

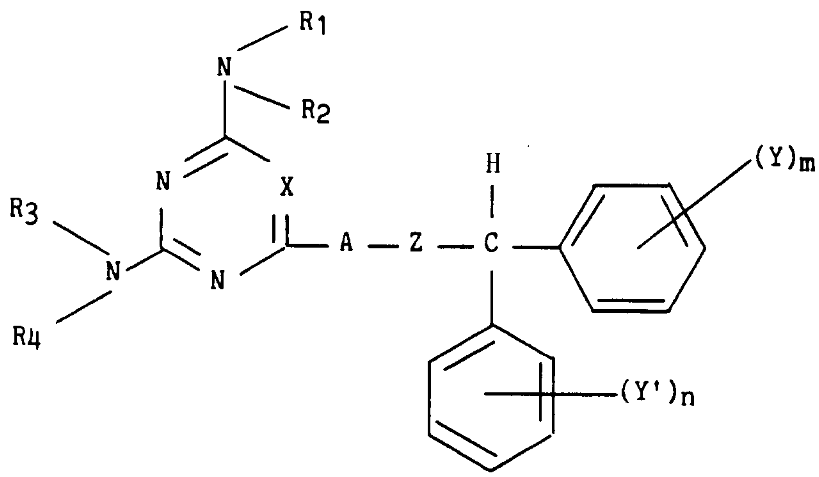

(I)

dans laquelle :

**a) X** représente le groupe CH ou un atome d'azote ;

**b) A** représente un groupe polyméthylène-imine de formule :

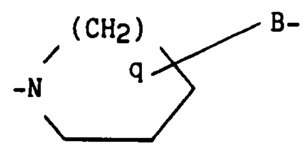

dans laquelle :

- **B** est un atome de soufre ou un radical NR' dans lequel R' représente un atome d'hydrogène ou un radical méthyle,
  et
  **q** est 2 ;

**c)** $R_1$, $R_2$, $R_3$, $R_4$ qui sont identiques ou différents, représentent chacun :

- un atome d'hydrogène, un radical allyle ou un radical propyle, étant entendu que l'un au moins des groupes

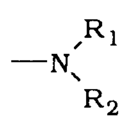

et

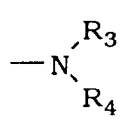

représente un radical allylamino ;

**d) Z** représente un radical méthylène ou éthylène ;

**e) Y** et **Y'**, qui sont identiques ou différents, représentent chacun un atome d' hydrogène ou de fluor, ou radical méthyle ou methoxy ;

**f) m** et **n**, qui sont identiques ou différents, représentent chacun les nombres entiers 1 ou 2 ;

et, les diastéréoisomères et énantiomères correspondants.

**2.** Les sels d'addition acides des dérivés de la revendication 1.

**3.** Les sels selon la revendication 2 qui sont physiologiquement tolérables.

**4.** La (bis allylamino-4,6 s.triazinyl-2)-1[(bis p.fluorophényl)-2,2 éthyl] amino-4 pipéridine.

**5.** La (bis allylamino-4,6 s.triazinyl-2)-1[(diméthoxy-3,5 phényl)-2 (méthyl-2 phényl)-2 éthyl amino]-4 pipéridine.

**6.** Le procédé de préparation des dérivés de la revendication 1, caractérisé en ce que l'on condense :

- soit une polyméthylène-imine de formule générale II :

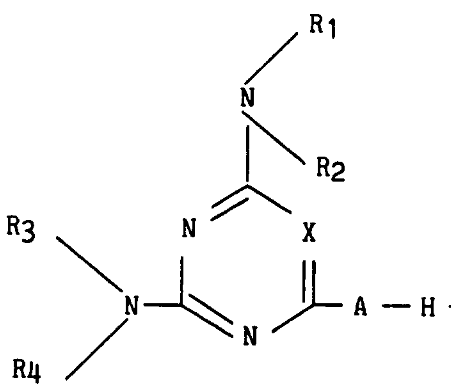

(II)

dans laquelle X, A, $R_1$, $R_2$, $R_3$ et $R_4$ ont les significations définies dans la revendication 1, sur un dérivé de formule générale III :

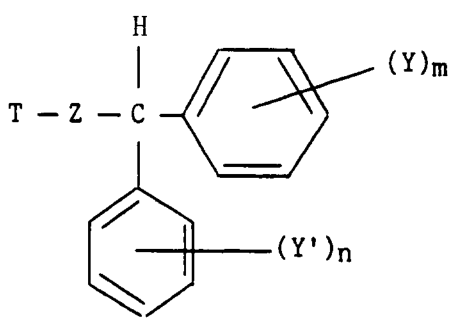

(III)

dans laquelle Z, Y, Y', m et n ont les significations définies dans la revendication 1 et T représente un atome d'halogène tel que par exemple un atome de chlore ou de brome ou un reste tosyloxy

- soit un dérivé halogéné de formule générale IV :

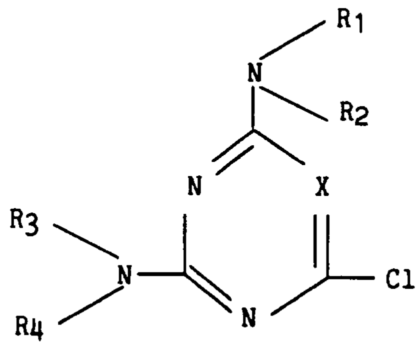

(IV)

dans laquelle X, $R_1$, $R_2$, $R_3$ et $R_4$ ont les significations définies dans la revendication 1, sur une polyméthylène-imine de formule générale V :

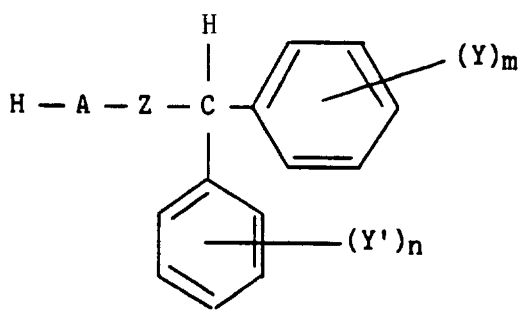

(V)

dans laquelle A, Z, Y, Y', m et n ont les significations définies dans la revendication 1.

**7.** Le procédé de préparation des dérivés de la revendication 1 répondant à la formule générale I' :

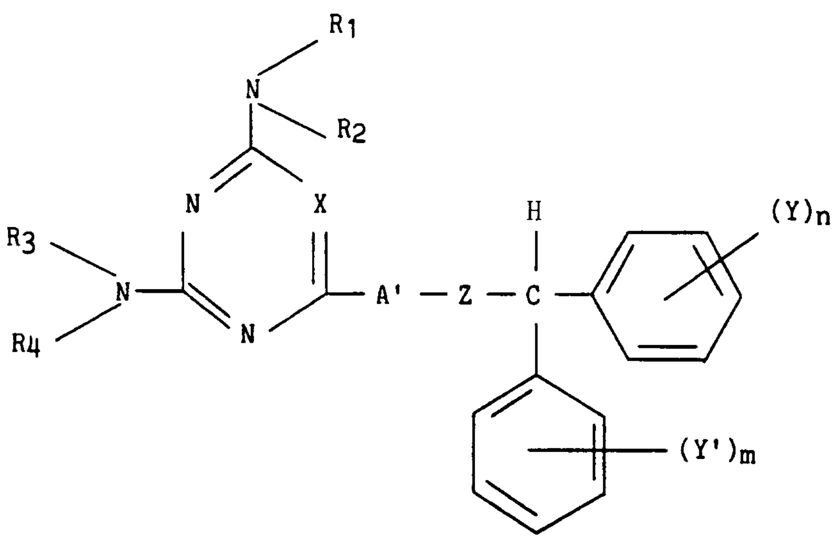

(I')

dans laquelle X, Z , $R_1$, $R_2$, $R_3$, $R_4$, Y, Y', m et n ont les significations définies dans la revendication 1 et A' représente un groupe polyméthylène-imine de formule :

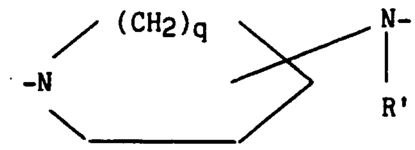

dans laquelle **q** et **R'** ont les significations définies dans la revendication 1, caractérisé en ce que l'on traite par le cyanoborohydrure de sodium un mélange de :

- la cétone de formule générale VI :

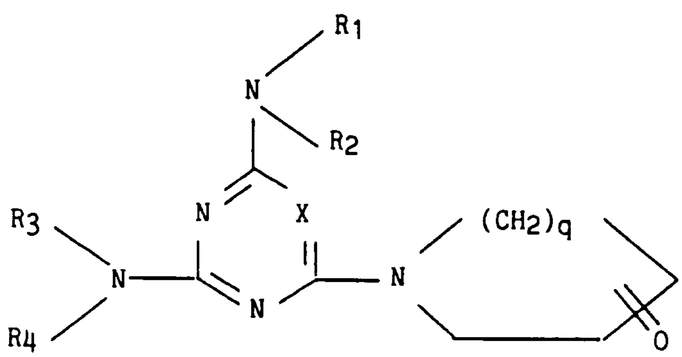

**(VI)**

dans laquelle X, $R_1$, $R_2$, $R_3$, $R_4$ et q ont les significations définies dans la revendication 1,

- et de l'amine de formule générale VII :

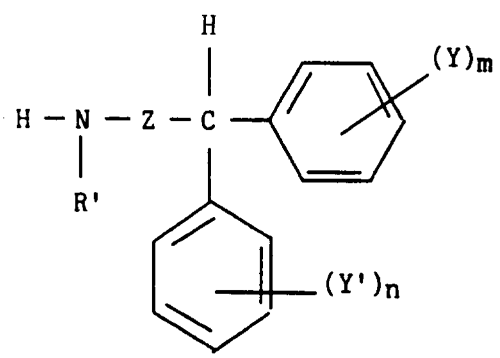

**(VII)**

dans laquelle R', Z, Y, Y', m et n ont les significations définies dans la revendication 1.

**8.** Les compositions pharmaceutiques contenant comme principe actif un dérivé selon les revendications 1 et 3 à 5, avec des excipients pharmaceutiques appropriés.

**9.** Les compositions pharmaceutiques selon la revendication 8 présentées sous une forme convenant notamment pour supprimer la résistance des cellules tumorales aux agents anti-cancéreux.

## Claims

**1.** Polymethyleneimines of the general formula I:

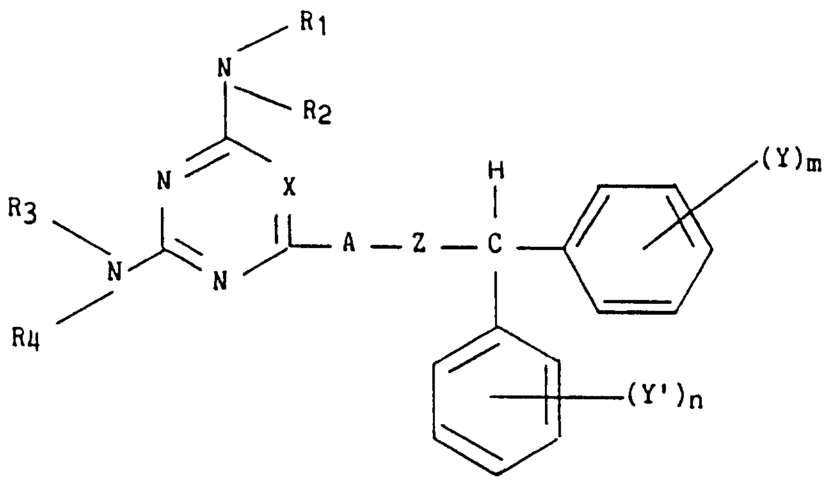

(I)

wherein:

a) X represents the group CH or a nitrogen atom;

b) A represents a polymethyleneimine group of the formula

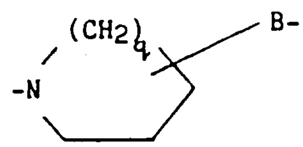

wherein:

- B is a sulphur atom or a radical NR' wherein R' represents a hydrogen atom or a methyl radical, and q is 2;

c) each of $R_1$, $R_2$, $R_3$ and $R_4$, which are identical or different, represents:

- a hydrogen atom, an allyl radical or a propyl radical, with the proviso that at least one of the groups

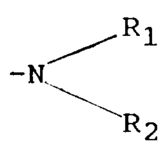

and

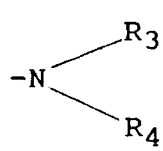

represents an allylamino radical;

d) Z represents a methylene or ethylene radical;

e) each of Y and Y', which are identical or different, represents a hydrogen or fluorine atom or a methyl or methoxy radical;

f) each of m and n, which are identical or different, represents the integer 1 or 2;

and the corresponding diastereoisomers and enantiomers.

**2.** The acid addition salts of the compounds of claim 1.

**3.** The salts according to claim 2 that are physiologically tolerable.

**4.** 1-(4,6-bis-allylamino-2-s-triazinyl)-4-[2,2-bis-(p-fluorophenyl)-ethylamino]-piperidine.

**5.** 1-(4,6-bis-allylamino-2-s-triazinyl)-4-[2-(3,5-di-methoxyphenyl)-2-(2-methylphenyl)-ethylamino]-piperidine.

**6.** Process for the preparation of the compounds of claim 1, characterised in that:

- either a polymethyleneimine of the general formula II:

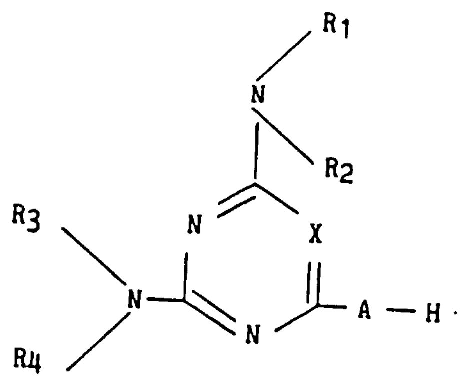

(II)

wherein X, A, $R_1$, $R_2$, $R_3$ and $R_4$ are as defined in claim 1, is condensed with a compound of the general formula III:

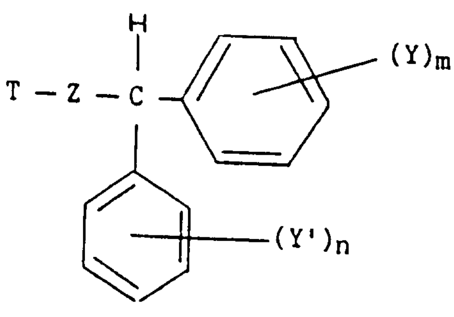

(III)

wherein Z, Y, Y', m and n are as defined in claim 1 and T represents a halogen atom, such as, for example, a chlorine or bromine atom, or a tosyloxy radical,

- or a halogen compound of the general formula IV:

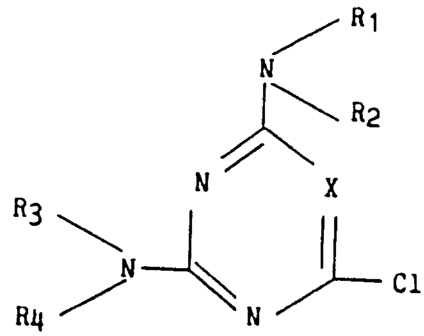

(IV)

wherein X, $R_1$, $R_2$, $R_3$ and $R_4$ are as defined in claim 1, is condensed with a polymethyleneimine of the general formula V:

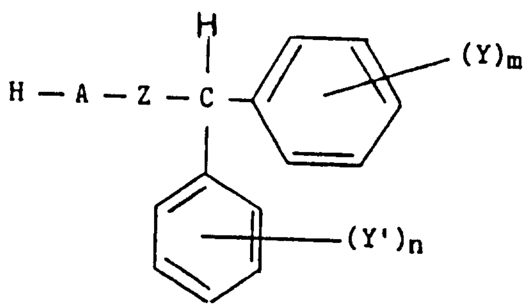

(V)

wherein A, Z, Y, Y' m and n are as defined in claim 1.

7. Process for the preparation of the compounds of claim 1 corresponding to the general formula I':

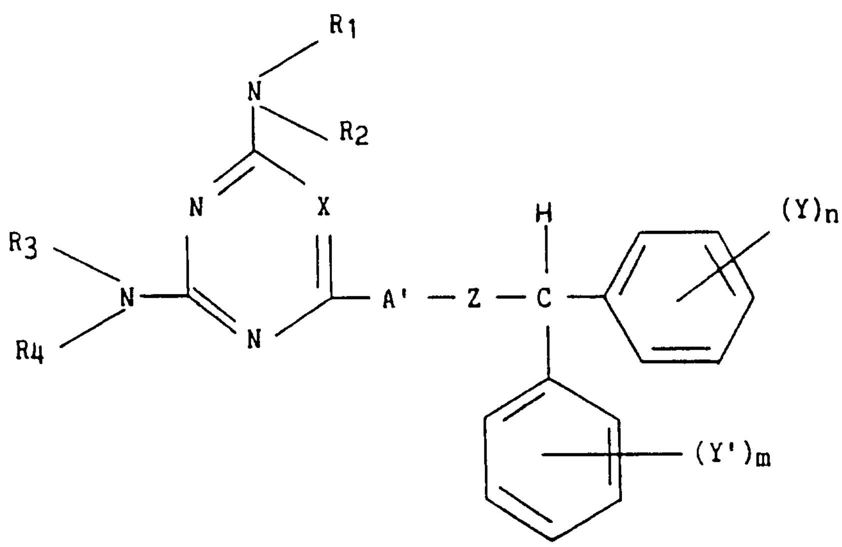

(I')

wherein X, Z, $R_1$, $R_2$, $R_3$, $R_4$, Y, Y', m and n are as defined in claim 1 and A' represents a polymethyleneimine group of the formula:

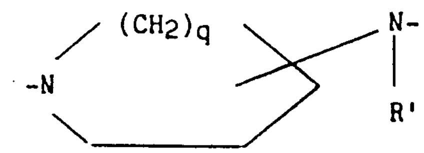

wherein q and R' are as defined in claim 1, characterised in that a mixture of:

- the ketone of the general formula VI:

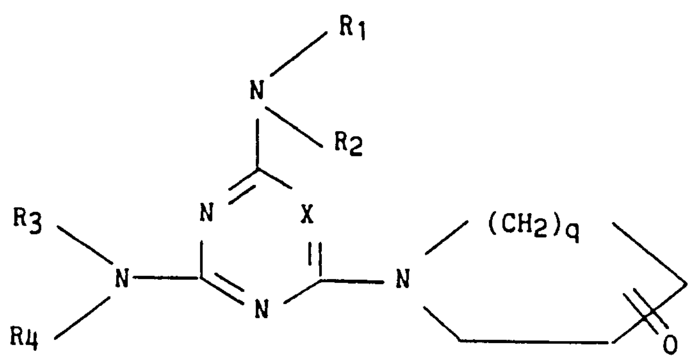

(VI)

wherein X, $R_1$, $R_2$, $R_3$, $R_4$ and q are as defined in claim 1,

- and the amine of the general formula VII:

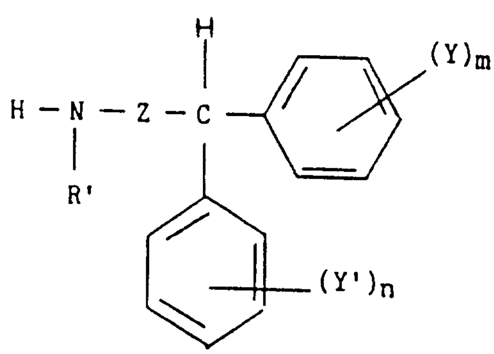

(VII)

wherein R', Z, Y, Y', m and n are as defined in claim 1, is treated with sodium cyanohorohydride.

**8.** Pharmaceutical compositions containing as active ingredient a compound according to claims 1 and 3 to 5, with pharmaceutically acceptable excipients.

**9.** Pharmaceutical compositions according to claim 8, presented in a form suitable especially for suppressing the resistance of tumour cells to anti-cancer agents.

## Patentansprüche

**1.** Polymethylenimine der allgemeinen Formel I:

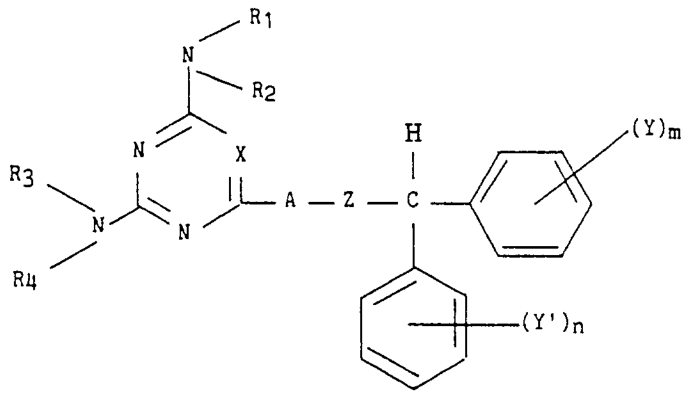

(I)

in der

a) X eine Gruppe CH oder ein Stickstoffatom;

b) A eine Polymethylenimingruppe der Formel:

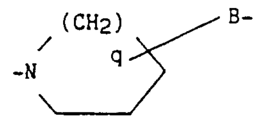

in der

- B ein Schwefelatom oder eine Gruppe NR', worin R' ein Wasserstoffatom oder eine Methylgruppe, und
  q 2 darstellen;

c) $R_1$, $R_2$, $R_3$, $R_4$, die gleichartig oder verschieden sein können, Jeweils

- ein Wasserstoffatom, eine Allylgruppe oder eine Propylgruppe mit der Maßgabe, daß mindestens eine der Gruppen

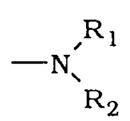 und 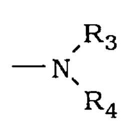

eine Allylaminogruppe darstellt;

d) Z eine Methylengruppe oder Ethylengruppe;

e) Y und Y', die gleichartig oder verschieden sein können, jeweils ein Wasserstoffatom oder ein Fluoratom oder eine Methylgruppe oder eine Methoxygruppe; und

f) m und n, die gleichartig oderverschieden sein können, jeweils ganze Zahlen mit den Werten von 1 oder 2 bedeuten;

und die entsprechenden Diastereoisomeren und Enantiomeren.

**2.** Additionssalze der Derivate nach Anspruch 1.

**3.** Salze nach Anspruch 2, die physiologisch verträglich sind.

**4.** 1-(4,6-Bisallylamino-s-triazin-2-yl)-4-[2,2-(bis-p-fluorophenyl)-ethylamino]-piperidin.

5. 1-(4,6-Bisallylamino-s-triazin-2-yl)-4-[2-(3,5-dimethoxy-phenyl)-2-(2-methyl-phenyl)-ethylamino]-piperidin.

6. Verfahren zur Herstellung der Derivate nach Anspruch 1, **dadurch gekennzeichnet**, daß man
- entweder ein Polymethylenimin der allgemeinen Formel II:

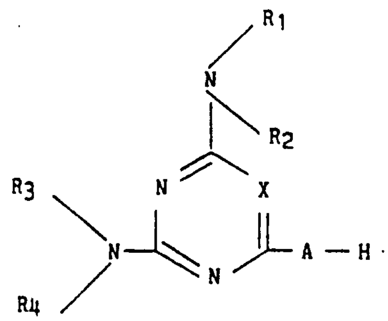

(II)

in der X, A, $R_1$, $R_2$, $R_3$ und $R_4$ die in Anspruch 1 angegebenen Bedeutungen besitzen, mit einem Derivat der allgemeinen Formel III:

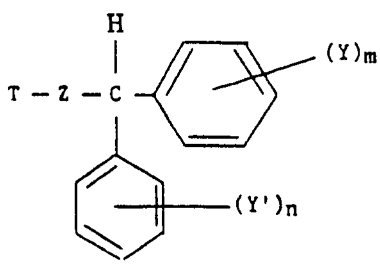

(III)

in der Z, Y, Y', m und n die in Anspruch 1 angegebenen Bedeutungen besitzen und T ein Halogenatom, wie beispielsweise ein Chloratom oder ein Bromatom oder einen Tosyloxyrest darstellt,
- oder ein Halogenderivat der allgemeinen Formel IV:

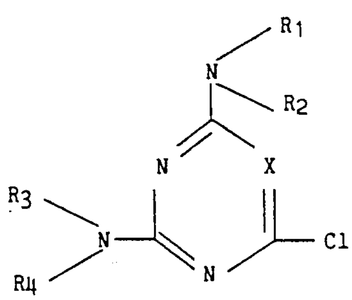

(IV)

in der X, $R_1$, $R_2$, $R_3$ und $R_4$ die In Anspruch 1 angegebenen Bedeutungen besitzen, mit einem Polymethylenimin der allgemeinen Formel V:

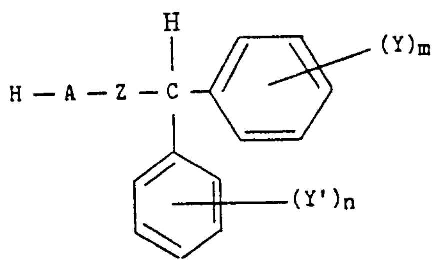

(V)

in der A, Z, Y, Y', m und n die in Anspruch 1 angegebenen Bedeutungen besitzen, kondensiert.

**7.** Verfahren zur Herstellung der Derivate nach Anspruch 1 der allgemeinen Formel I':

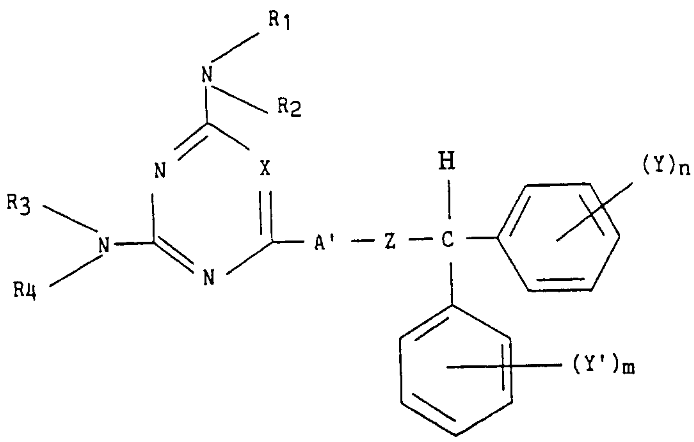

(I')

in der X, Z, $R_1$, $R_2$, $R_3$, $R_4$, Y, Y', m und n die in Anspruch 1 angegebenen Bedeutungen besitzen und A' eine Polymethyleniminggruppe der Formel:

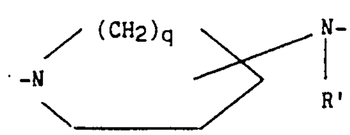

darstellt, worin q und R' die in Anspruch 1 angegebenen Bedeutungen besitzen, **dadurch gekennzeichnet**, daß man eine Mischung aus

- dem Keton der allgemeinen Formel VI:

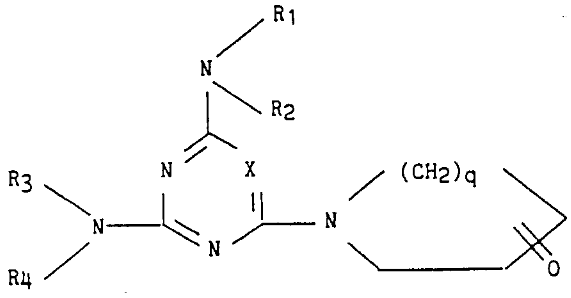

(VI)

in der X, $R_1$, $R_2$, $R_3$, $R_4$ und q die in Anspruch 1 angegebenen Bedeutungen besitzen, und

- dem Amin der allgemeinen Formel VII:

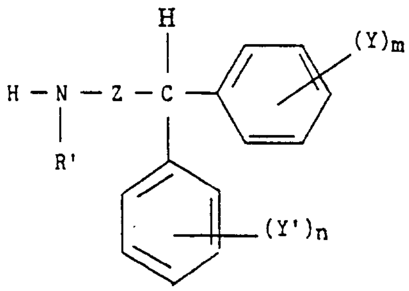

(VII)

in der R', Z, Y, Y', m und n die in Anspruch 1 angegebenen Bedeutungen besitzen, mit Natriumcyanoborhydrid behandelt.

**8.** Pharmazeutische Zubereitungen enthaltend als Wirkstoff ein Derivat nach den Ansprüchen 1 und 3 bis 5 zusammen mit geeigneten pharmazeutischen Trägermaterialien.

**9.** Pharmazeutische Zubereitungen nach Anspruch 8 in einer Form, die insbesondere dazu geeignet ist, die Resistenz von Tumorzellen gegen Antikrebsmittel zu unterdrücken.